# EUROPÄISCHE PATENTSCHRIFT

(11) **EP 0 578 796 B1**
(45) Veröffentlichungstag und Bekanntmachung des Hinweises auf die Patenterteilung: **14.01.1998**
(21) Anmeldenummer: 93902024.4
(22) Anmeldetag: 04.02.1993
(51) Int. Cl.: A61M 35/00

(54) **EINRICHTUNG ZUR ABGABE EINES ARZNEIMITTELS**
DEVICE FOR DISPENSING A MEDICAMENT
INSTALLATION DE DISTRIBUTION D'UN MEDICAMENT

(30) Priorität: 04.02.1992 CH 309/92
(43) Veröffentlichungstag der Anmeldung: 19.01.1994
(73) Patentinhaber: ASULAB S.A., CH-2502 Bienne (CH)
(72) Erfinder: STRAUSAK, Sabina, CH-4056 Basel (CH); LEUENBERGER, Hans, CH-4148 Pfeffingen (CH)
(74) Vertreter: Caron, Gérard
(86) Internationale Anmeldenummer: CH9300030
(87) Internationale Veröffentlichungsnummer: WO9314808

(56) Entgegenhaltungen:
- EP-A- 0 336 543
- WO-A-91/03271
- DE-A- 2 902 183
- FR-A- 2 562 800

## Beschreibung

### Gebiet der Erfindung

Die Erfindung betrifft eine Einrichtung zur Abgabe eines Arzneimittels nach dem Oberbegriff von Anspruch 1, und wie aus der WO 91/03271 bekannt.

Die Einrichtung ist insbesondere vorgesehen, um ein Arzneimittel während einer relativ langen, zum Beispiel mindestens einige Tage betragenden Zeitdauer abzugeben und einem Organismus zu spenden. Die Einrichtung kann zum Beispiel zur Anordnung an der Hautoberfläche eines menschlichen oder eventuell tierischen Körpers vorgesehen sein, um diesem von dessen Aussenseite her ein Arzneimittel transdermal zuzuführen. Die Einrichtung kann jedoch auch in einen Körper eines Menschen oder Tieres implantierbar sein, um beispielsweise ein Arzneimittel an die in einem Gewebe und/oder in einem Hohlraum des Körpers vorhandene Flüssigkeit abzugeben.

### Stand der Technik

Bekannte Einrichtungen zum Abgeben eines Arzneimittels besitzen einen Speicher mit einem Speicherraum, der eine das gelöste Arzneimittel aufweisende Lösung enthält. Der Speicherraum ist mindestens zum Teil durch eine Membran begrenzt, durch welche das Medikament hindurchdiffundieren kann. Einrichtungen dieser Art können für die transdermale Zufuhr des Arzneimittels oder für die Implantation in einem Körper eines Menschen oder Tieres vorgesehen sein.

Derartige Einrichtungen geben das Arzneimittel bei der Benutzung kontinuierlich und unterbruchslos an bzw. in den zu behandelnden Organismus ab, bis der Speicher leer ist. Die Abgabe des Arzneimittels erfolgt dabei mit einer Rate, die häufig entweder fortlaufend abnimmt oder während eines grossen Teils des Abgabevorgangs ungefähr konstant ist und dann zum Beispiel in der Endphase des Abgabevorgangs abnimmt. Eine solche kontinuierliche Abgabe ist jedoch bei vielen Arzneimitteln aus verschiedenen Gründen ungünstig. Zum Beispiel kann bei verschiedenen Arzneimitteln deren Bedarf und/oder deren Aufnahme sowie Verarbeitung in einem Organismus zeitlich periodisch, wie zum Beispiel im Tagesrythmus, oder aperiodisch, zum Beispiel infolge exogener Einflüsse, variieren. Ferner kann ein Organismus bei länger dauernder, konstanter Zufuhr eines Arzneimittels, wie beispielsweise Nitroglycerin, sogenannte Toleranzen - d.h. Gewöhnungserscheinungen - entwickeln, welche die Wirksamkeit des Arzneimittels reduzieren, so dass entweder eine Abnahme der therapeutischen Wirkung in Kauf genommen oder die Zufuhrrate des Arzneimittels im Verlauf der Behandlung erhöht werden muss.

Bei der WO 91/03271 ist eine Einrichtung gezeigt, bei welcher der Membranquerschnitt von aussen mittels eines wegnehmbaren Schlüssels eingestellt und der jeweils geforderten Arzneimittel-Zufuhrrate angepasst werden kann.

Es sind auch Einrichtungen bekannt, um ein Arzneimittel einem Organismus transdermal mit Hilfe der Iontophorese zuzuführen. Derartige Einrichtungen besitzen eine elektrische Batterie, eine Elektronikvorrichtung und Elektroden. Mit diesen kann ein elektrisches Feld erzeugt werden, das einen in den Körper des Organismus eindringenden, Ionen des Arzneimittels enthaltenden elektrischen Strom erzeugt, so dass das Arzneimittel als Ionenstrom in den zu behandelnden Körper gelangt. Der Ionenstrom kann wahlweise kontinuierlich oder intermittierend zugeführt werden.

Die Iontophorese hat jedoch den Nachteil, dass sie nur für Arzneimittel anwendbar ist, die aus Ionen bestehen bzw. solche bilden können. Zudem müssen die Ionen unter der Einwirkung des benötigten elektrischen Feldes stabil bleiben. Ferner besteht bei der Iontophorese die Gefahr, dass das angelegte elektrische Feld und der erzeugte elektrische Strom ein unangenehmes Kribbeln, Reizungen und Erärmungen oder sogar Verbrennungen und/oder eventuell sonstige Schädigungen des Organismus verursachen.

Andere bekannte Arzneimittel-Abgabe-Einrichtungen besitzen einen Speicher sowie eine elektrisch antreibbare Pumpe. Solche Einrichtungen erlauben zwar je nach Bedarf eine kontinuierliche sowie konstante oder eine intermittierende Abgabe eines Arzneimittels, werden jedoch nur für die Implantation in einen Körper und nicht für die transdermale Zufuhr eines Arzneimittels benutzt. Die bekannten, eine Pumpe aufweisenden Einrichtungen wären für die transdermale Arzneimittel-Zufuhr auch gar nicht geeignet, weil sie nur eine kleine Auslassöffnung für das Arzneimittel haben, so dass dieses kaum auf einen für eine transdermale Zufuhr ausreichend grossen Bereich einer Hautoberfläche verteilt werden könnte. Zudem können die Förderraten der gegenwärtig erhältlichen Pumpen nicht beliebig klein gemacht werden, so dass es in den meisten Fällen erforderlich ist, das Arzneimittel oder - genauer gesagt - dessen Wirkstoff in stark verdünnter Form in den zu behandelnden Organismus zu pumpen. Dies hat unter anderem den Nachteil, dass entsprechend grosse Speicher benötigt werden. Zudem verbraucht eine elektrisch antreibbare Pumpe während ihres Betriebs dauernd elektrische Energie, so dass Einrichtungen mit einer Pumpe mit einer relativ grossen Batterie ausgerüstet werden müssen.

### Darstellung der Erfindung

Der Erfindung liegt die Aufgabe zugrunde, eine Einrichtung zur Abgabe eines Arzneimittels zu schaffen, die ermöglicht, Nachteile der bekannten Einrichtungen zu vermeiden. Dabei soll insbesondere ausgehend von den bekannten Einrichtungen mit einem Speicher und mit einer für das gespeicherte Arzneimittel durchlässigen Membran ermöglicht werden, die Abgabe des Arzneimittels in gewünschter Weise zeitlich zu steuern und zum Beispiel zeitweise zu sperren oder mindestens zu reduzieren und danach wieder freizugeben bzw. zu vergrössern.

Diese Aufgabe wird gemäss der Erfindung durch die zusätzlichen Merkmale des Anspruchs 1 gelöst

Besonders vorteilhafte Ausgestaltungen der Erfindung gehen aus den abhängigen Ansprüchen hervor.

Das im Speicherraum der Einrichtung gespeicherte, bei der Benutzung der Einrichtung abzugebende, d.h. zu spendende und einem Organismus zuzuführende Arzneimittel ist mindestens zum Teil in einem ebenfalls im Speicherraum vorhandenen, fliessfähigen, nämlich flüssigen oder eventuell gelartigen Lösungsmittel gelöst. Das Arneimittel kann zum Beispiel aus Nitroglyzerin, Estradiol, Scopolamin, Nikotin, Salbutamol, einem Analgetikum, wie Indomethacin, einem Antirheumatikum oder einem andern Wirkstoff bestehen. Das Lösungsmittel kann zum Beispiel hydrophob sein und aus einem biokompatiblen, natürlichen und/oder synthetischen Öl - wie Rhizinusöl oder Miglyol - bestehen. Das Lösungsmittel kann jedoch auch hydrophil sein oder aus einer Mischung mit mindestens einer hydrophoben und mindestens einer hydrophilen Komponente bestehen.

Der Speicherraum der Einrichtung enthält also vor deren Benutzung ein Speicher- und/oder Füllmaterial, das mindestens zum Teil aus einer Lösung besteht. Das Arzneimittel kann zum Beispiel vor dem Abgabevorgang und beim Beginn von diesem vollständig im Lösungsmittel gelöst sein. Das Speicher- und/oder Füllmaterial kann jedoch vor der Arzneimittel-Abgabe sowie beim Beginn von dieser zusätzlich zu gelöstem Arzneimittel noch ungelöstes Arzneimittel aufweisen. Dieser ungelöste Anteil des gespeicherten Arzneimittels kann zum Beispiel in der Form einer Vielzahl kleiner, fester Teilchen und/oder eventuell flüssiger Tröpfchen vorliegen, die im Lösungsmittel dispergiert - d.h. suspendiert bzw. emulgiert - und mehr oder weniger gleichmässig verteilt sind. Der ursprünglich ungelöste Anteil des Arzneimittels kann jedoch auch in Form einer kompakten Schicht gespeichert sein, die beispielsweise an der Innenfläche einer zur Begrenzung des Speicherraums dienenden Wand haftet. Zudem besteht die Möglichkeit, den Speicherraum durch eine gelochte Trennwand oder dergleichen in zwei Bereiche zu unterteilen, von denen der eine als Kompartiment zum Aufnehmen des ursprünglich ungelösten, festen, beispielsweise körnigen Arzneimittel-Anteils dient, während der andere Speicherraumbereich das Arzneimittel dann ausschliesslich in gelöstem Zustand enthält. Wenn der Speicherraum zusätzlich zu gelöstem Arzneimittel noch ungelöstes Arzneimittel enthält, kann selbstverständlich - bezogen auf das Volumen des Speicherraums - eine grössere Arzneimittelmenge gespeichert werden als wenn der Speicherraum ausschliesslich gelöstes Arzneimittel enthält.

Die Membran besitzt gemäss der Erfindung mindestens einen Durchlassbereich, der für das von der Einrichtung abzugebende Arzneimittel durchlässig ist. Das letztere kann dementsprechend durch den mindestens einen Durchlassbereich der Membran hindurchdiffundieren. Die Membran und das Lösungsmittel sind vorzugsweise derart aneinander und an die Art des Arzneimittels angepasst, dass die Membran für das Lösungsmittel im wesentlichen undurchlässig ist. Die Membran soll dabei entweder überhaupt kein Lösungsmittel oder höchsten so wenig Lösungsmittel durchlassen, dass bei einem Arzneimittel-Abgabevorgang ein grosser Teil des Lösungsmittels im Speicherraum verbleibt, bis die vorgesehene Menge des Arzneimittels abgegeben ist. Dabei sollen vorzugsweise mindestens 50 Gew.% des Lösungsmittels und zweckmässiger mindestens 75 Gew.% und noch besser mindestens 90 Gew.% des Lösungsmittels im Speicherraum verbleiben, bis mindestens 50 Gew.% und zum Beispiel mindestens 75 Gew.% oder noch besser mindestens 90 Gew.% der anfänglich gespeicherten Menge des Arzneimittels abgegeben ist.

Die Membran soll aus einem gegen das Arzneimittel und das Lösungsmittel resistenten Material bestehen und ihrerseits das Arzneimittel nicht schädigen. Wenn die Einrichtung für die transdermale Zufuhr des Arzneimittels vorgesehen ist, soll die Membran zudem für die Haut des zu behandelnden Menschen oder Tieres gut verträglich sein und also weder Reizungen noch Schädigungen der Haut verursachen. Wenn die Einrichtung zur Implantation in einen Körper eines Menschen oder Tieres vorgesehen ist, soll die Membran - wie übrigens auch alle andern, äussere Begrenzungsflächen der Einrichtung bildenden Teile von dieser - biokompatibel sein und keine toxischen Wirkungen haben. Die Membran kann zum Beispiel aus einer Folie bzw. einem Film aus einem hydrophoben oder hydrophilen, porösen Membranmaterial - zum Beispiel Kunststoff - bestehen. Die im Membranmaterial vorhandenen Poren haben vorzugsweise eine weniger als 1 Mikrometer betragende, effektive Grösse, wobei die letztere der Maschenweite eines Siebes entspricht und die maximale Grösse von durchgelassenen Teilchen angibt. Für die Bildung von Membranen geeignete, poröse, aus Polypropylen bestehende, flexible Folien bzw. Filme sind zum Beispiel unter den Marken- sowie Typenbezeichnungen CELGARD 2400 und CELGARD 2500 von der Hoechst Celanese Corporation, Charlotte, NC 28217, USA, erhältlich. Diese Folien oder Filme sind ungefähr 0,025 mm dick und haben Poren mit einer effektiven Grösse von etwa 0,05 bzw. 0,075 Mikrometern.

Die Membran ist vorzugsweise derart mit den anderen Teilen der Einrichtung verbunden, dass mindestens ein ihren Durchlassbereich bzw. ihre Durchlassbereiche enthaltender Abschnitt glatt sowie faltenfrei ist und dass die Aussenfläche des genannten Abschnitts der Membran einen beispielsweise ebenen Abschnitt einer fiktiven Hüllfläche bildet, welche die Einrichtung umhüllt, sich mindestens stellenweise an die Einrichtung anschmiegt und ausschliesslich aus ebenen und/oder konvex gekrümmten Abschnitten besteht. Die Einrichtung wird für die Benutzung vorzugsweise derart am oder im mit dem Arzneimittel zu versorgenden Körper eines Patienten oder Tieres angeordnet, dass sich der bzw. jeder Durchlassbereich der Membran bei dauernd oder zumindest häufig eingenommenen Lagen des Patienten bzw. Tieres unter dem Speicherraum und/oder bei dessen tiefster Stelle befindet.

Das gemäss der Erfindung vorhandene, verstellbare und/oder verformbare Steuerelement der Einrichtung ermöglicht den Zugang des Arzneimittels vom Speicherraum zum mindestens einen Durchlassbereich der Membran zu verändern. Das Steuerelement kann eine dem Absperrelement und/oder Drosselelement eines Ventils und/oder Hahns entsprechende Funktion ausüben.

Das Steuerelement kann zum Beispiel aus einem verstellbaren Schieber - wie einem Drehschieber oder eventuell einem entlang einer Geraden verschiebbaren Schieber - bestehen. Der Schieber kann mindestens einen Durchlassbereich aufweisen, in welchem mindestens ein Durchlassloch und zum Beispiel mehrere Durchlasslöcher oder sogar sehr viele sowie sehr kleine, aus durchgehenden Poren bestehende Durchlasslöcher vorhanden sind. Das Steuerelement kann jedoch eventuell auch piezoelektrisch verformbar sein.

Das Steuerelement kann zum Beispiel wahlweise in einen Freigabe-Zustand oder in einen Sperr-Zustand gebracht werden. Unter dem Freigabe-Zustand wird dabei diejenige Stellung und/oder Form des Steuerelements verstanden, bei welcher das Arzneimittel den grösstmöglichen Zugang zum mindestens einen Durchlassbereich der Membran hat. Mit dem Sperr-Zustand ist diejenige Stellung und/oder Form des Steuerelements gemeint, bei welcher der Zugang des Arzneimittels gesperrt oder zumindest auf den kleinstmöglichen Wert gedrosselt ist. Hier soll noch der Fall näher betrachtet werden, dass das Steuerelement den Zugang des Arzneimittels zur Membran im Sperr-Zustand nicht vollständig sperrt und nur drosselt. In diesem Fall soll die Abgaberate des Arzneimittels im Sperr-Zustand höchstens 40% und noch besser höchstens 30% der sich bei der gleichen Konzentration des gelösten Arzneimittels im Freigabe-Zustand ergebenden Abgaberate betragen. Wenn nun das Steuerelement zum Beispiel abwechselnd während gewissen Freigabe-Zeitintervallen in den Freigabe-Zustand und während gewissen Sperr-Zeitintervallen in den Sperr-Zustand gebracht wird und wenn die Konzentration des gelösten Arzneimittels in solchen aufeinanderfolgenden Zeitintervallen ändert, kann man die sich während mindestens zwei aufeinanderfolgenden Freigabe-Zeitintervallen ergebenden Werte der Abgaberate näherungsweise durch eine Freigabe-Approximationskurve oder Freigabe-Approximationsgerade darstellen, welche sich mindestens über ein Sperr-Zeitintervall erstreckt. Ferner kann man die sich im Sperr-Zustand ergehenden Werte der Abgaberate in analoger Weise näherungsweise durch eine Sperr-Approximationskurve oder Sperr-Approximationsgerade darstellen. Der in einem bestimmten Zeitpunkt durch die Sperr-Approximationskurve oder -Approximationsgerade dargestellte Näherungswert soll dann vorzugsweise höchstens 40% und noch besser höchstens 30% des im betreffenden Zeitpunkt durch die Freigabe-Approximationskurve oder -Approximationsgerade dargestellten Werts betragen.

Man kann auch noch die Möglichkeit vorsehen, das Steuerelement in einen Zwischen-Zustand oder wahlweise in einen von mehreren verschiedenen Zwischen-Zuständen zu bringen. In einem solchen Zwischen-Zustand kann die Abgaberate des Arzneimittels dann einen Wert haben, der zwischen den sich im Freigabe-Zustand und im Sperr-Zustand ergebenden Werten liegt.

Falls der Speicherraum bei der Abgabe von Arzneimittel ausschliesslich gelöstes Arzneimittel enthält, nimmt die Konzentration des in der Lösung vorhandenen Arzneimittels zuerst in der Nähe der Membran und danach auch in weiter von dieser entfernten Bereichen ab. In der Lösung bildet sich daher ein Konzentrationsgefälle aus. Wenn das Steuerelement vom Sperr-Zustand in den Freigabe-Zustand gebracht wird und anschliessend während einer gewissen Zeitdauer in diesem bleibt, steigt dementsprechend die Arzneimittel-Abgaberate zuerst sprungartig an und nimmt danach wieder ab. Wenn das Steuerelement anschliessend wieder in den Sperr-Zustand gebracht und die Abgabe dadurch unterbrochen oder mindestens stark reduziert wird, nimmt auch das Konzentrationsgefälle in der Lösung wieder ab, so dass die Konzentration des gelösten Arzneimittels wieder mehr oder weniger homogen wird. Falls das Steuerelement im Sperr-Zustand den Zugang des Arzneimittels zur Membran nicht vollständig sperrt, kann die Arzneimittel-Konzentration der Lösung auch während des Sperr-Zustandes abnehmen.

Falls der Speicherraum sowohl gelöstes als auch ungelöstes Arzneimittel enthält und das Steuerelement vom Sperr-Zustand in den Freigabe-Zustand gebracht wird, nimmt die Arzneimittel-Konzentration zwar mindestens in einem an die Membran angrenzenden Bereich der Lösung ebenfalls ab. Während des Abgabevorgangs wird jedoch vorher ungelöstes Arzneimittel gelöst. Nach einer gewissen Zeitdauer kann sich daher ein ungefähr stationärer Zustand einstellen, in welchem die Arzneimittel-Abgaberate ungefähr konstant bleibt, bis das Steuerelement wieder in den Sperr-Zustand gebracht wird.

Wenn der Speicherraum ungelöstes Arzneimittel enthält, ändert also die Arzneimittel-Abgaberate bei sich im Freigabe-Zustand befindendem Steuerelement weniger, als wenn der Spreicherraum ausschliesslich gelöstes Arzneimittel enthält. Die Abgaberaten haben dann ferner in verschiedenen Freigabe-Zeitintervallen ungefähr gleich grosse Maximalwerte, was für viele Verwendungen vorteilhaft ist.

Das verstellbare und/oder verformbare Steuerelement ist vorzugsweise möglichst nahe bei der Membran angeordnet und derart ausgebildet sowie bemessen, dass in seinem Sperr-Zustand höchstens ein sehr geringer Teil der insgesamt in der Einrichtung vorhandenen Menge der Lösung in Kontakt mit dem Durchlassbereich bzw. den Durchlassbereichen der Membran steht. Dies trägt dazu bei, dass der zeitliche Verlauf der Abgaberate den Änderungen des Zustands des Steuerelements insbesondere auch dann relativ rasch folgt, wenn dieses vom Freigabe-Zustand in den Sperr-Zustand gebracht wird.

Der Speicherraum der Einrichtung ist vorzugsweise allseitig durch eine dichte Wandung sowie das Steuerelement und/oder die Membran gegen die Umgebung der Einrichtung abgeschlossen. Ferner ist der Speicherraum vorzugsweise vor der Benutzung der Einrichtung durch das mindestens zum Teil aus einer Lösung bestehende Speicher- und/oder Füllmaterial vollständig ausgefüllt. Wenn die Arzneimittel-Konzentration der im Speicherraum vorhandenen Lösung beim Abgeben von Arzneimittel abnimmt, kann das Volumen der Lösung unter Umständen im Verlauf der Zeit ebenfalls abnehmen. Falls das im Speicherraum vorhandene Speicher- und/oder Füllmaterial vor der Benutzung der Einrichtung zusätzlich zu gelöstem Arzneimittel noch ungelöstes Arzneimittel aufweist und dieses bei der Benutzung in Lösung geht und danach abgegeben wird, verkleinert sich das Volumen des Speicher- und/oder Füllmaterials ungefähr um das ursprünglich vom ungelösten Arzneimittel eingenommene Volumen.

Die Einrichtung kann daher noch mit Kompensationsmitteln zur Kompensation von Volumenänderungen des Speicher- und/oder Füllmaterials ausgerüstet sein. Diese Kompensationsmittel können zum Beispiel ein an einer Wand des Speichers befestigtes und mindestens zum Teil an den Speicherraum angrenzendes oder vollständig in diesen angeordnetes, deformierbares Kompensationselement aufweisen, das sein Volumen derart ändern kann, dass es sich bei abnehmendem Volumen des Speicher- und/oder Füllmaterials ausdehnt und das freiwerdende Volumen ausfüllt. Das Kompensationselement kann zum Beispiel einen geschlossene Poren enthaltenden Schaumgummikörper aufweisen, der vor der Benutzung der Einrichtung durch das Speicher- und/oder Füllmaterial komprimiert wird und sich dann bei abnehmendem Volumen des Speicher- und/oder Füllmaterials ausdehnt. Das Kompensationselement kann statt dessen auch durch einen Beutel oder Ballon gebildet sein, der eine flexible, beispielsweise elastisch dehnbare Hülle und einen allseitig von dieser umschlossenen Hohlraum besitzt, der mit einem zum Beispiel aus Luft bestehenden Gas gefüllt ist, dessen Druck grösser als der in der Umgebung der Einrichtung herrschende Luftdruck ist. Eine andere Möglichkeit besteht darin, einen Teil der Begrenzung des Speicherraums durch eine flexible Folie zu bilden, die durch mindestens ein auf ihrer Aussenseite angeordnetes, etwa aus einer Feder oder einem Schaumgummikörper bestehendes Druckelement gegen das Innere des Speicherraums gedrückt wird. Des weiteren besteht die Möglichkeit, einen Teil der Begrenzung des Speicherraums durch eine gummielastische, dehnbare Hülle, oder einen Federbalg zu bilden. Die Hülle bzw. der Balg kann dann vor der Benutzung der Einrichtung derart vorgespannt - d.h. gedehnt - werden, dass sie bzw. er bestrebt ist, sich bei der Abgabe von Arzneimittel zusammenzuziehen.

Dadurch, dass der Speicherraum vor der Benutzung der Einrichtung vollständig mit Speicher- und/oder Füllmaterial gefüllt wird und dass eine allfällige, bei der Arzneimittelabgabe stattfindende Verkleinerung des Volumens des Speicher- und/oder Füllmaterials durch Kompensationsmittel kompensiert wird, kann gewährleistet werden, dass die Lösung bei sich im Freigabe-Zustand befindendem Steuerelement unabhängig von der Lage der Einrichtung in Kontakt mit der Membran gelangt.

Das Steuerelement ist elektrisch steuerbar, d.h. mit elektrisch steuerbaren Stellmitteln verbunden oder versehen. Diese Stellmittel können zum Beispiel einen elektrischen Motor oder mindestens ein piezoelektrisches Element und Elektroden aufweisen. Insbesondere wenn man zulässt, dass das Steuerelement in der Sperr-Stellung die Arzneimittel-Abgabe nicht vollständig sperrt, sondern nur in der bereits beschriebenen Weise drosselt, kann die Einrichtung derart ausgebildet werden, dass das Verstellen und/oder Verformen des Steuerelements nur wenig Energie verbraucht. Dies ergibt unter anderem den Vorteil, dass die zum Verstellen und/oder Verformen des Steuerelements erforderliche Energie von einer eine relativ kleine Kapazität besitzenden Batterie geliefert werden kann.

Die Einrichtung besitzt elektronische, elektrisch leitend mit den Stellmitteln verbundene Schaltungsmittel, mit denen das Steuerelement automatisch gesteuert und zum Beispiel abwechselnd vom Sperr-Zustand in den Freigabe-Zustand und wieder in den Sperr-Zustand gebracht werden kann. Dabei kann zum Beispiel vorgesehen sein, dass die elektronischen Schaltungsmittel das Steuerelement periodisch ein Mal oder mehrere Male pro Periode während einer beispielsweise 30 Minuten bis einige Stunden betragenden Freigabe-Zeitdauer in den Freigabe-Zustand bringen. Wenn das Steuerelement während einer Periodendauer mehrmals in den Freigabe-Zustand gebracht wird, kann ferner vorgesehen werden, dass die verschiedenen Freigabe-Zustände verschiedene Zeitabstände voneinander haben und/oder unterschiedlich lange dauern. Die Periodendauer kann zum Beispiel einige Stunden oder einen ganzen Tag oder sogar mehrere Tage oder Wochen dauern.

Die Einrichtung kann zudem mindestens ein manuell betätigbares Bedienungsorgan aufweisen, das einem Patienten oder einer Pflegeperson im Bedarfsfall die Möglichkeit gibt, die Periodendauer und/oder die Freigabe-Zeitdauer einzustellen und/oder das Steuerelement statt automatisch manuell zu steuern.

Das Steuerelement der Einrichtung ermöglicht also, das Arzneimittel intermittierend abzugeben oder zumindest die Abgaberate abwechselnd deutlich zu verkleinern und wieder zu vergrössern. Dadurch kann die Arzneimittelabgabe gut an die Art des Arzneimittels und an einen zeitlich ändernden Bedarf eines Patienten oder Tieres angepasst werden, dem das Arzneimittel gespendet wird. Ferner kann die Entwicklung von Toleranzen verhindert oder zumindest stark reduziert werden. Aus diesen Gründen kann auch die insgesamt zu verabreichende Arzneimittelmenge reduziert werden. Dies ergibt wiederum den Vorteil, dass unerwünschte Nebenwirkungen vermindert oder sogar ganz vermieden werden können.

Das als mögliches Arzneimittel genannte Nitroglycerin kann zum Beispiel einem an Angina pectoris leidenden Patienten transdermal in einem Tageszyklus zugeführt werden. Dabei kann das Steuerelement bei der normalen Behandlung automatisch einmal oder mehrmals täglich vorübergehend in den Freigabe-Zustand gebracht werden. Wenn nun der Patient zum Beispiel bei sich im Sperr-Zustand befindendem Steuerelement merkt, dass er einen Anfall bekommt, kann er das Steuerelement durch Betätigen des vorzugsweise vorhandenen Bedienungsorgans in den Freigabe-Zustand bringen. Eventuell kann auch vorgesehen werden, dass das Steuerelement beim normalen, automatisch gesteuerten Betrieb statt in den Freigabe-Zustand jeweils nur in einen Zwischen-Zustand gebracht wird und dass das Steuerelement nur durch manuelles Betätigen des Bedienungsorgans in den Freigabe-Zustand gebracht werden kann.

Beim ebenfalls als mögliches Arzneimittel erwähnten Estradiol handelt es sich um ein auch vom weiblichen Körper selbst produziertes Hormon, wobei die natürliche Produktionsrate im Menstruationszyklus und eventuell zusätzlich im Tagesrythmus ändert. Bei einer zur Abgabe von Estradiol dienenden Einrichtung kann das Steuerelement dementsprechend zum Beispiel derart gesteuert werden, dass das Estradiol periodisch ändernd mit einer Periodendauer abgegeben wird, die einer mittleren Menstruationsperiodendauer entspricht. Das Steuerelement kann zu diesem Zweck zum Beispiel in jeder Periode während mehreren aufeinanderfolgenden Tagen - etwa während 5 bis 10 Tagen - dauernd im Sperr-Zustand bleiben und im restlichen Teil der Periode einmal oder mehrmals täglich während einer gleich bleibenden oder im Verlauf einer Periodendauer variierenden Freigabe-Zeitdauer in den Freigabe-Zustand gebracht werden. Falls pro Tag mindestens zweimal Estradiol abgegeben wird, kann die Abgabe- oder Freigabe-Zeitdauer bei den verschiedenen, während des gleichen Tages erfolgenden Abgaben gleich oder verschieden festgelegt werden.

Bei einer für die transdermale Zufuhr eines Arzneimittels vorgesehenen Einrichtung kann diese bei einer vorteilhaften Ausgestaltung zwei Bänder aufweisen, deren eine Enden an einem zur Einrichtung gehörenden Support und/oder Gehäuse befestigt sind. Die anderen Endabschnitte der Bänder können dann mit Verschlussmitteln - zum Beispiel Klett-Verschlussmitteln - versehen sein, durch welche die beiden Bänder lösbar miteinander verbunden werden können. Derartige Bänder ermöglichen, die Einrichtung zum Beispiel lösbar an einem Arm oder Bein eines Menschen oder Tieres zu befestigen. Eine derartige Befestigungsweise hat den Vorteil, dass sie keine Klebstoffe erfordert, die unter Umständen Allergien oder sonstige Hautreizungen verursachen könnten.

Es besteht jedoch auch die Möglichkeit, eine für die transdermale Arzneimittel-Zufuhr vorgesehene Einrichtung mit mindestens einem Klebband zu befestigen. Die Einrichtung kann dann nicht nur an einem Arm oder Bein, sondern auch am Thorax oder an irgend einem andern Körperteil befestigt werden. Zudem kann die Einrichtung dann eventuell durch das Klebband bzw. durch die Klebbänder vollständig abgedeckt und wasserdicht gegen die Umgebung abgeschlossen werden, so dass ein die Einrichtung tragender Patient sich auch die diese umgebenden Bereiche seiner Körperoberfläche problemlos waschen oder sogar baden und duschen kann.

### Kurze Beschreibung der Zeichnung

Der Erfindungsgegenstand wird nun anhand in der Zeichnung dargestellter Ausführungsbeispiele näher erläutert. In der Zeichnung zeigt
die Fig. 1 einen schematisierten Schnitt einer Einrichtung zur Abgabe eines Arzneimittels mit einem drehbaren, sich in der Sperr-Stellung befindenden Steuerelement,
die Fig. 2 einen entlang der Linie II - II der Fig. 1 verlaufenden Schnitt durch die Einrichtung, wobei ein Teil des Steuerelements weggebrochen ist,
die Fig. 3 einen Ausschnitt aus der Fig. 1 in grösserem Massstab, wobei das mit der Verzahnung des Steuerelements kämmende Zahnrad ebenfalls geschnitten ist,
die Fig. 4 einen andern Ausschnitt aus der Fig. 1 bei sich im Sperr-Zustand befindendem Steuerelement im gleichen Massstab wie die Fig. 3,
die Fig. 5 einen der Fig. 4 entsprechenden Ausschnitt aus der Einrichtung, aber bei sich im Freigabe-Zustand befindendem Steuerelement,
die Fig. 6 einen schematischen Querschnitt durch einen Körperteil eines Menschen und eine Ansicht der am Körperteil befestigten Einrichtung in kleinerem Massstab als in den Figuren 1 bis 5,
die Fig. 7 ein Diagramm mit einer den zeitlichen Verlauf der Arzneimittel-Abgabe der Einrichtung darstellenden Messkurve,
die Fig. 8 einen ungefähr der Fig. 3 entsprechenden, schematischen Ausschnitt aus einer andern Einrichtung mit einem drehbaren Steuerelement,
die Fig. 9 einen Schnitt durch die zum Teil in der Fig. 8 dargestellten Einrichtung entlang der Linie IX - IX der Fig. 8 in kleinerem Massstab als diese,
die Fig. 10 einen schematischen Ausschnitt aus noch einer anderen Einrichtung mit einem piezoelektrisch verformbaren, sich in der Sperr-Stellung befindenden Steuerelement und
die Fig. 11 einen Ausschnitt aus der in der Fig. 10 gezeichneten Einrichtung, aber mit sich in der Freigabe-Stellung befindendem Steuerelement.

### Bevorzugte Ausführungsbeispiele der Erfindung

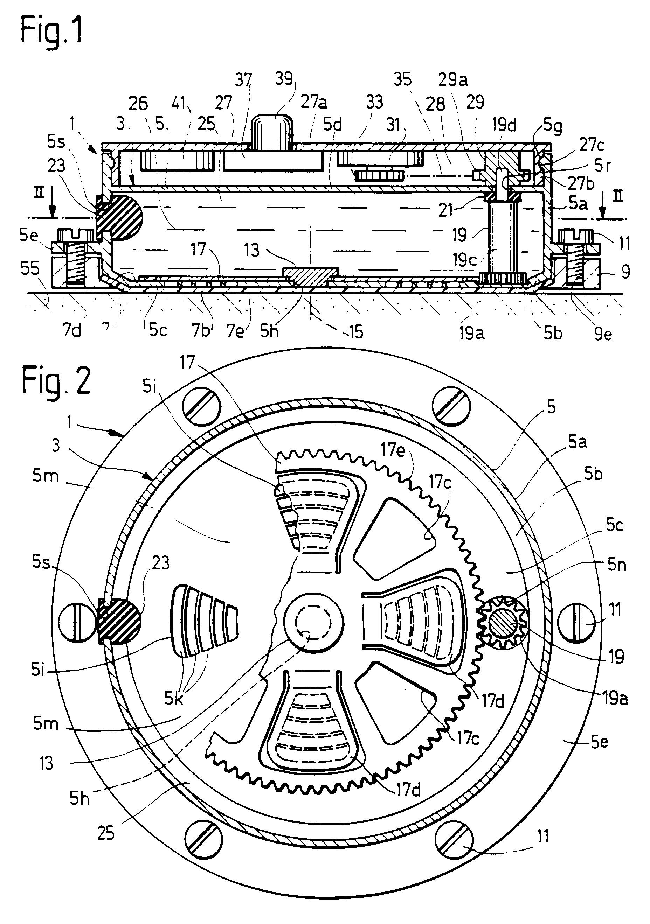

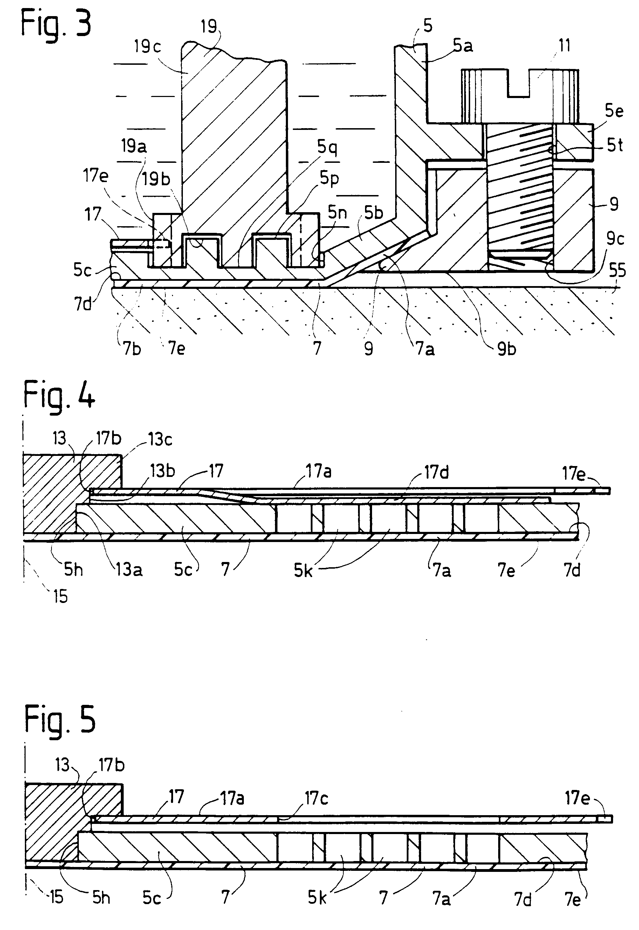

In den Figuren 1, 2 und 6 ist eine als Ganzes mit 1 bezeichnete Einrichtung zur Abgabe eines Arzneimittels ersichtlich. Die Einrichtung 1 besitzt einen Speicher 3 mit einem zum Teil auch in den Figuren 3, 4 sowie 5 gezeichneten Speichergehäuse 5. Dieses ist im allgemeinen rotationssymmetrisch zu einer nicht gezeichneten Achse und besitzt eine im wesentlichen formfeste Wandung mit einem zylindrischen Mantel 5a. Dieser ist an seinem sich in der Fig. 1 unten befindenden Ende über einen nach unten zur Achse des Speicherbehälters hin geneigten, konischen Wandabschnitt 5b mit einer ersten ebenen, radialen Wand 5c verbunden, die bei der in der Fig. 1 gezeichneten Lage der Einrichtung den Boden des Speichergehäuses 5 bildet. Der Mantel 5a ist in der Nähe seines anderen, sich in der Fig. 1 oben befindenden Endes mit einer zweiten ebenen, radialen, als Decke dienenden Wand 5d verbunden. Das Speichergehäuse 5 besitzt ferner einen vom Mantel 5a weg radial nach aussen ragenden Flansch 5e. Der in der Fig. 1 nach oben über die zweite Wand 5d vorstehende Abschnitt des Mantels 5a ist mit mindestens einem nach innen ragenden Rastvorsprung 5g - nämlich mit einer umlaufenden Rippe oder mit einigen entlang dem Mantelumfang verteilten Nocken - versehen. Das die vorgängig beschriebenen Teile aufweisende Speichergehäuses ist in der Fig. 1 schematisch als einstückiger Körper gezeichnet, besteht aber in Wirklichkeit aus mindestens zwei ursprünglich separaten und dann unlösbar oder lösbar miteinander verbundenen Teilen.

Die erste Wand 5c ist mit einem ein wenig exzentrisch angeordneten, besonders deutlich in den Figuren 4 und 5 ersichtlichen Loch 5h versehen. Die erste Wand 5c besitzt ferner mindestens einen Durchlassbereich 5i, vorzugsweise mindestens zwei und nämlich vier gleichmässig um das Loch 5h herum verteilte, besonders deutlich in der Fig. 2 ersichtliche, etwa kreissektorförmige Durchlassbereiche 5i. Die Wand 5c ist bei jedem Durchlassbereich 5i mit mindestens einem die erste Wand 5c durchdringenden Loch 5k und nämlich mit fünf schlitzförmigen Löchern 5k versehen. Diese sind durch schmale Stege voneinander getrennt und verlaufen gleich wie die letzteren entlang von Kreisbogen um das Zentrum des Lochs 5h herum. Zwischen jedem Paar von entlang einem das Loch 5h umschliessenden Kreis aufeinander folgenden Durchlassbereichen 5i ist ein kompakter - d.h. lochfreier - Sperrbereich 5m vorhanden. Die Sperrbereiche 5m erstrecken sich dabei über einen grösseren Zentriwinkel als die Durchlassbereiche 5i. Die innere, obere Fläche der ebenen Wand 5c ist in der Nähe ihrer am weitesten vom Loch 5h entfernten Umfangsstelle mit einer besonders deutlich in der Fig. 3 ersichtlichen, kreisringförmigen Ausnehmung 5n - d.h. Ringnut - versehen, die einen Zapfen 5p umschliesst. Dieser ragt ein wenig über die innere, obere Fläche des restlichen Teils der ersten Wand 5c hinaus und ist im Zentrum mit einem Sackloch 5q versehen.

Die zweite ebene Wand 5d besitzt ein durchgehendes Loch 5r, dessen Zentrum in einer zur Achse des Speichergehäuses parallelen Richtung mit dem Zentrum der Ausnehmung 5n und des Sacklochs 5q fluchtet. Der Mantel 5a ist mit einem Loch 5s versehen. Ferner enthält der Flansch 5e entlang seinem Umfang verteilte Löcher 5t.

Eine poröse, für das abzugebende Arzneimittel durchlässige, flexible, aussen am Speichergehäuse 5 befestigte Membran 7 besitzt einen an der äusseren, konischen Fläche des konischen Wandabschnitts 5b anliegenden Randbereich 7a und einen ebenen Durchlassbereich 7b, der an der äusseren, unteren Fläche der ersten ebenen Wand 5c anliegt. Die an dieser anliegende Innenfläche des Membran-Durchlassbereichs 7b ist mit 7d bezeichnet. Die Aussenfläche des Membran-Durchlassbereichs ist mit 7e bezeichnet. Die konische Aussenfläche des Wandabschnitts 5b und der Randbereich 7a der Membran 7 sind vorzugsweise höchstens 45° und noch besser höchstens 30° gegen die erste ebene Wand 5c geneigt, so dass die Membran 7 und insbesondere deren Durchlassbereich 7b glatt und faltenlos bleibt.

Ein zur Befestigung der Membran 7 dienender Klemmring 9 besitzt einen den Mantel 5b unterhalb des Flansches 5e umschliessenden Abschnitt und einen von diesem nach innen ragenden Vorsprung 9a, der den Randbereich 7a der Membran 7 am konischen Wandabschnitt 5b festklemmt. Wie es besonders deutlich in der Fig. 3 ersichtlich ist, befindet sich die untere, radiale und ebene Begrenzungsfläche 9b des Klemmrings 9 oberhalb der Aussenfläche 7e des ebenen Durchlassbereichs 7b der Membran 7. Die ebene Membran-Aussenfläche 7e bildet in der in den Figuren 1 und 3 bis 5 gezeichneten Lage der Einrichtung 1 dem tiefstgelegenen Abschnitt einer aus ebenen und konvex gekrümmten Abschnitten gebildeten Hüllfläche der Einrichtung. Der Klemmring ist mit Gewindebohrungen 9c versehen und wird durch Schrauben 11, welche die Löcher 5t durchdringen und in die Gewindebohrungen 9c eingeschraubt sind, am Speichergehäuse befestigt.

Ein besonders deutlich in den Figuren 4 und 5 ersichtliches Lager 13 besitzt einen fest und mindestens einigermassen, dicht im Loch 5h sitzenden, zum Beispiel durch Einpressen und/oder Vernieten in diesem befestigten Zapfen 13a, eine Erweiterung 13b mit einer an der inneren, oberen Fläche der ersten Wand 5c anliegenden Schulterfläche und einen in kleinem Abstand von der ersten Wand 5c stehenden Kopf 13c. Das Lager 13 definiert eine zur Achse des Speichergehäuses 5 parallele, exzentrische Achse 15.

Ein Steuerelement 17 besteht aus einer im allgemeinen kreisförmigen Scheibe und besitzt einen ebenen Hauptabschnitt 17a. Dieser ist im Zentrum mit einem kreisförmigen Loch 17b versehen, dessen Rand die Erweiterung 13b des Lagers 13 umschliesst, wie es besonders deutlich in den Figuren 4 und 5 ersichtlich ist. Das Steuerelement 17 wird durch das Lager 13 radial sowie axial gelagert, so dass es um die Achse 15 herum gedreht oder hin und her verschwenkt werden kann. Das Steuerelement 17 besitzt mindestens ein Durchlassloch 17c sowie mindestens einen Sperrbereich 17d, wobei nämlich je vier um die Achse 15 herum verteilte Durchlasslöcher 17c und Sperrbereiche 17d vorhanden sind. Die Durchlasslöcher 17c haben in einer zur Achse 15 parallelen Projektion ungefähr oder genau die gleiche Form wie die stückweise durch Ränder der Löcher 5k definierte Umrissform der Durchlassbereiche 5i. Jeder Sperrbereich 17d des Steuerelements 17 besteht aus einer federnden Zunge, die zusammen mit den restlichen Teilen des Steuerelements aus einem einstückigen Körper gebildet ist. Das sich am nächsten bei der Achse 15 befindende Ende der Zunge bildet deren Wurzel und hängt mit dem ebenen, das Loch 17a sowie die Durchlasslöcher 17c begrenzenden Hauptabschnitt 17a des Steuerelements zusammen. Die freien Ränder jeder Zunge sind durch einen entlang von ihnen verlaufenden Schlitz vom restlichen Steuerelement getrennt. Jede Zunge hat bei ihrer Wurzel einen vom Hauptabschnitt 17a weg nach aussen sowie nach unten - d.h. zur ersten ebenen Wand 5c hin - geneigten und zum Beispiel abgekröpften Abschnitt, wie es in der Fig. 4 gezeichnet ist. Die gegen die erste Wand 5c hin versetzten Abschnitte der Zungen können federnd an der inneren Fläche der ersten ebenen Wand 5c anliegen. Der Hauptabschnitt 17a des vom Lager 13 mit kleinem axialen Spiel gelagerten Steuerelements 17 wird durch die Federwirkung der Zungen so weit von der Wand 5c abgehoben, bis er an der der ersten Wand 5c zugewandten, radialen Fläche des Kopfes 13c ansteht. Das Steuerelement 17 ist entlang seinem Umfang mit einer Verzahnung 17e versehen.

Wenn sich das Steuerelement 17 gemäss den Figuren 1, 2 und 4 im Sperr-Zustand bzw. in der Sperr-Stellung befindet, bedeckt der zur ersten Wand 5c hin versetzte und an dieser anliegende Abschnitt jedes aus einer Zunge bestehenden Sperrbereichs 17d des Steuerelements 17 einen Durchlassbereich 5i der ersten Wand 5c. Wie man in der Fig. 2 sehen kann, ragt jeder von einer Zunge gebildete Sperrbereich 17d in einer zur Achse 15 parallelen Projektion allseitig ein wenig über die zum betreffenden Durchlassbereich 5i gehörenden Löcher 5k hinaus, so dass er diese Löcher 5k mehr oder weniger dicht abschliesst.

Wenn das Steuerelement 17 ausgehend von seinem Sperr-Zustand bzw. seiner Sperr-Stellung um einen Winkel von 45° um die Achse 15 herum geschwenkt wird, gelangt es in seinen Freigabe-Zustand bzw. in seine Freigabe-Stellung. Wie es in der Fig. 5 gezeichnet ist, befindet sich dann jedes Durchlassloch 17c des Steuerelements 17 über den Löchern 5k vom einen der Durchlassbereiche 5i der ersten Wand 5c. Im FreigabeZustand des Steuerelements 17 steht also jedes Durchlassloch 17c mindestens zum Teil und zum Beispiel vollständig sowie genau in Deckung mit dem betreffenden Durchlassbereich 5i.

Das Steuerelement 17 bildet also einen Drehschieber. Ein zum Drehen und/oder Verschwenken des Steuerelements dienendes Übertragungselement 19 besitzt ein mit der Verzahnung 17b des Steuerelements 17 kämmendes Zahnrad 19a. Dessen Durchmesser und Zähnezahl sind dabei wesentlich kleine als der Durchmesser bzw. die Zähnezahl des Steuerelements 17. Das Zahnrad 19a hat bei der sich in den Figuren 1 und 3 unten befindenden Stirnseite eine in der Fig. 3 ersichtliche Ausnehmung 19b, nämlich eine Ringnut. Der diese umschliessende Zahnkranz des Zahnrads 19a ragt in axialer Richtung in die ringförmige Ausnehmung 5n der ersten Wand 5c hinein. Dementsprechend ragt deren ringförmiger, hohlzylinderförmiger Zapfen 5p in die Ausnehmung 19b des Zahnrads 19a hinein, so dass dieses an der ersten Wand 5c radial gelagert und axial abgestützt ist. Das Übertragungselement 19 besitzt eine starr mit dem Zahnrad 19a verbundene, zum Beispiel mit diesem zusammen aus einem einstückigen Körper bestehenden Welle 19c. Diese ragt durch das Loch 5r der zweiten ebenen Wand 5d hindurch und ist dort gelagert Die Welle 19c ist oberhalb der zweiten Wand 5d mit einem Kupplungsabschnitt 19d versehen, dessen Zweck noch erläutert wird. Die Durchführung der Welle 19c durch die zweite Wand 5d ist durch eine Dichtung 21 abgedichtet.

Das Speichergehäuse 5, der Klemmring 9, das Steuerelements 17 und das Antriebselement 19 bestehen zum Beispiel aus einem metallischen Material, wie rostfreiem Stahl oder Titan. Die Membran 7 besteht zum Beispiel aus einer der in der Einleitung angegebenen, unter der Bezeichnung CELGARD 2400 bzw. CELGARD 2500 erhältlichen Folien. Es ist noch anzumerken, dass die Figuren 1 bis 6 zur Verbesserung der Klarheit teilweise nicht massstäblich gezeichnet sind. Dabei wurde insbesondere die Membran 7 und zum Teil auch das Steuerelement 17 in übertriebener Dicke gezeichnet. Der Aussendurchmesser des Mantels 5a kann zum Beispiel etwa 3 cm bis 4 cm betragen. Die erste ebene Wand 5c kann zum Beispiel ungefähr 0,5 mm dick sein. Die Membran 7 kann eine Dicke von 0,025 mm haben. Die Materialdicke des Steuerelements 17 kann zum Beispiel 0.07 mm bis bis 0,2 mm betragen. Das axiale Spiel des Steuerelements zwischen der Membran 7 und dem Kopf des Lagers 13 beträgt zum Beispiel höchstens oder ungefähr 0,01 mm.

Das Loch 5s ist durch ein Verschlusselement 23 dicht abgeschlossen. Dieses dient als Septum, besteht aus einem gummieleastischen Material und ist derart ausgebildet, dass das Loch 5s und das Verschlusselement 23 von einer hohlen Nadel durchstochen werden können und das Verschlusselement 23 das Loch 5c nach dem Herausziehen der Nadel wieder dicht abschliesst. Der freie, d.h. der nicht vom Lager 13, Steuerelement 17, Antriebselement 19 sowie Verschlusselement 23 eingenommene Bereich des Innenraumes des Speichers 3 bildet dessen Speicherraum 25. Dieser wird vor der Benutzung der Einrichtung mit einem Speicher- und/oder Füllmaterial 26 gefüllt, das gemäss der Einleitung mindestens zum Teil aus einer Lösung besteht. Das Verschlusselement 23 kann eventuell noch zusätzlich als Kompensationselement dienen, um in der in der Einleitung beschriebenen Weise Volumenänderungen des Speicher- und/oder Füllmaterials 26 zu kompensieren.

Ein Support 27 besitzt einen zum Beispiel ebenen, scheibenförmigen Deckteil 27a und einen in der Fig. 1 axial von diesem weg nach unten in den sich über der zweiten Wand 5d des Speichergeäuses 5 befindenden Abschnitt des Mantels 5a hineinragenden Ringteil 27b. Dessen Aussenfläche besitzt mindestens eine Rastausnehmung 27c, nämlich eine Ringnut oder entlang dem Umfang des Ringteils verteilte Vertiefungen. Der mindestens eine Rastvorsprung 5g des Speichergehäuses 5 und die mindestens Rastausnehmung 27c des Supports 27 bilden zusammen Rast- und Verbindungsmittel, die den Support 27 bei der fertiggestellten und betriebsbereiten Einrichtung fest, aber lösbar mit dem Speichergehäuse 5 verbinden. Im übrigen dient der Support 27 auch als Teil eines von ihm zusammen mit dem Speichergehäuse 5 gebildeten Gehäuses.

In dem zwischen der Wand 5d des Speichergehäuses 5 und dem Deckteil 27a des Supports 27 vorhandenen Innenraum 28 ist ein nur schematisch gezeichnetes Kupplungselement 29 mit nicht gezeichneten Lagermitteln drehbar gelagert. Dieses bildet zusammen mit dem Kupplungsabschnitt 19d des Übertragungselements 19 eine lösbare Kupplung, so dass das Kupplungselement 29 beim Verbinden des Supports 27 mit dem Speichergehäuse 5 drehfest mit dem Übertragungselement 19 gekoppelt und beim Entfernen des Supports 27 vom Speichergehäuse 5 wieder vom Übertragungselement entkoppelt wird. Der Kupplungsabschnitt 19d und das Kupplungselement 29 besitzen zum Beispiel in gekoppeltem Zustand aneinander angreifende Mitnehmermittel. Das Kupplungselement 29 besitzt zum Beispiel ein Loch, in das der Kupplungsabschnitt 19d beim Verbinden des Supports 27 mit dem Speichergehäuse 5 hineingesteckt werden kann. Die Umfangsfläche des Kupplungsabschnitts 19d und die Begrenzungsfläche des im Kupplungselement 29 vorhandenen Lochs können dann zum Beispiel Abflachungen haben, welche die Mitnehmermittel bilden. Statt dessen können auch Mitnehmermittel mit mindestens einem lösbar in ein Loch oder in eine Nut eingreifenden Vorsprung oder Zapfen vorhanden sein. Das Kupplungselement 29 ist beispielsweise noch mit einem Zahnrad 29a versehen oder fest verbunden.

Ein elektrischer Motor 31 ist ebenfalls im Innenraum 28 angeordnet und am Support 27 befestigt. Der Motor 31 ist zum Beispiel in der Art eines für Armbanduhren verwendbaren Schrittschaltmotors ausgebildet. Ein auf der Welle des Motors 31 sitzendes und durch dessen drehbares Zahnrad 33 ist über ein schematisch durch eine strichpunktierte Linie angedeutetes Getriebe 35 mit dem Zahnrad 29a verbunden, könnte aber statt dessen direkt mit diesem in Eingriff stehen. Der elektrisch antreibbare und steuerbare Motor 31 bildet zusammen mit dem Zahnrad 33, dem Getriebe 35, dem Kupplungselement 29 und dem Übertragungselement 19 Stellmittel zum Verstellen des Steuerelements 17.

Der Motor 31 ist elektrisch leitend mit einer am Support 27 befestigten Elektronikvorrichtung 37 verbunden. Diese besitzt elektronische Schaltungsmittel zum Steuern - d.h. Einschalten und Ausschalten - des Motors 31. Die elektronischen Schaltungsmittel können zum Beispiel eine programmierbare, integrierte Schaltung aufweisen. Die Elektronikvorrichtung 37 ist mit mindestens einem Bedienungsorgan 39 versehen und/oder elektrisch verbunden. Dieses ist von der Umgebung der Einrichtung her manuell betätigbar und kann zum Beispiel durch eine Schaltvorrichtung mit einer Drucktaste gebildet sein, die durch ein im Deckteil 27a vorhandenes Loch hindurchragt. Das Bedienungsorgan 39 oder eines der Bedienungsorgane kann jedoch eventuell auch einen manuell verschwenk- oder drehbaren Knopf besitzen. Eine wie die Elektronikvorrichtung 37 im Innenraum 28 angeordnete und am Support 27 befestigte Batterie 41 ist elektrisch leitend mit der Elektronikvorrichtung 41 und über diese und/oder direkt mit dem Motor 31 verbunden.

Der Speicherraum 25 der Einrichtung 1 wird vor deren Benutzung mit dem das zu speichernde Arzneimittel enthaltenden Speicher- und/oder Füllmaterial 26 gefüllt. Die Membran 7 kann vor oder nach dem Füllen des Speicherraums 25 durch eine aufgeklebte und wegziehbare Folie oder durch andere lösbare Verschlussmittel dicht verschlossen werden, bis die Einrichtung benutzt wird. Wenn das Speicher- und/oder Füllmaterial 26 aus einer reinen Lösung oder aus einer feine, dispergierte Teilchen oder Tröpfchen enthaltenden Lösung besteht, kann der Speicherraum 25 während und/oder nach der Herstellung des Speichers 3 evakuiert werden. Danach kann das gesamte Speicher- und/oder Füllmaterial mit Hilfe einer hohlen, vorübergehend durch das Verschlusselement 23 hindurchgesteckten Nadel in den Speicherraum 25 eingebracht werden.

Falls das Speicher- und/oder Füllmaterial hingegen einen festen Anteil in der Form grosser Körner oder einer festen, an einer Innenfläche des Speichergehäuses haftende Schicht oder eines sonstigen festen und relativ grossen Körpers aufweist, kann diese feste Anteil des Speicher- und/oder Füllmaterials eventuell bereits in den Speicherraum 25 eingebracht werden, bevor dieser durch Verbinden von ursprünglich separaten Teilen des Speichergehäuses und/oder durch Einsetzen des Verschlusselements 23 gegen die Umgebung abgeschlossen wird. Nach dem Abschliessen des Speicherraums kann man diesen dann noch mit einer Lösung oder mit einem zur Bildung einer solchen dienenden Lösungsmittel auffüllen, die bzw. das mit einer hohlen Nadel durch das Verschlusselement 23 hindurch zugeführt wird.

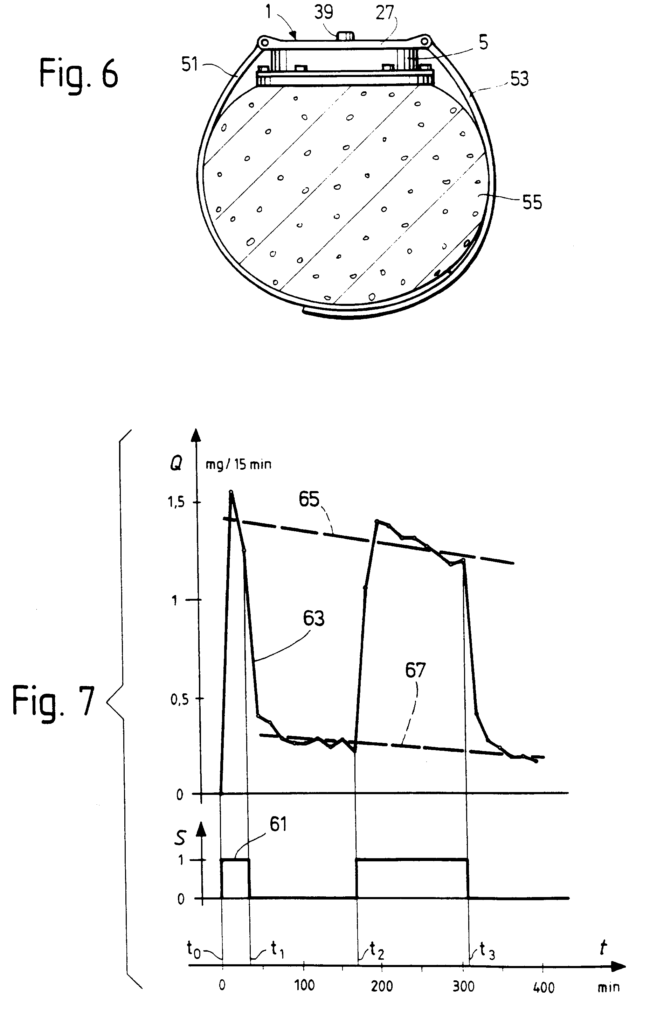

Zwei in der Fig. 6 ersichtliche Bänder 51, 53 sind bei ihren einen Enden am Support 27 befestigt. Die andern Endabschnitte der beiden Bänder 51, 53 sind mit Klettverschlussmitteln oder dergleichen versehen, so dass sie lösbar miteinander verbunden werden können.

Die Elektronikvorrichtung 37 kann bei der Herstellung der Einrichtung 1 ausgebildet und programmiert werden, um den Motor 31 bei der Benutzung der Einrichtung automatisch für die periodische Arzneimittel-Abgabe zu steuern. Es kann zudem vorgesehen werden, dass die Periodendauer der Abgabezyklen und/oder die Freigabe-Zeitdauer und/oder ein anderer Verfahrensparameter vor der Benutzung der Einrichtung individuell eingestellt werden kann. Dies kann zum Beispiel durch Betätigen des mindestens einen Bedienungsorgangs 39 oder mit Hilfe mindestens eines Stellorgans geschehen, das sich im Innenraum 28 befindet und durch Trennen des Supports 27 vom Speichergehäuse 5 zugänglich wird.

Für die Benutzung der Einrichtung 1 entfernt man die sie gegebenenfalls abdichtende Folie bzw. die sonst vorhandenen Verschlussmittel und befestigt die Einrichtung mit Hilfe der Bänder 51, 53 lösbar an einem zum Beispiel aus einem Arm bestehenden Körperteil 55 eines Menschen oder Tieres. Die Einrichtung 1 liegt dann mit der Aussenfläche 7e der Membran 7 fest an der Hautoberfläche des Körperteils 55 an. Danach dann die Einrichtung durch Betätigen des bzw. eines Bedienungsorgans 39 in Betrieb gesetzt werden, so dass die Elektronikvorrichtung 37 den Motor 31 in der vorgegebenen Weise steuert.

Bei der Benutung der Einrichtung kann der Motor 31 über das Übertragungselement 19 das Steuerelement 17 verschwenken bzw. drehen. Das Steuerelement 17 kann dabei von Zeit zu Zeit um einen Zentriwinkel von 45° verschwenkt und dadurch abwechselnd in den Sperr-Zustand und den Freigabe-Zustand gebracht werden. Eventuell kann das Steuerelement auch in einen Zwischen-Zustand bzw. in eine Zwischen-Stellung gebracht werden, in dem bzw. der die als Sperrbereiche 17d dienenden Zungen die Löcher 5k der Durchlassbereiche 5i nur teilweise bedecken und abschliessen.

Während der Benutzung der Einrichtung gibt diese vorher im Speicher- und/oder Füllmaterial 26 gelöstes Arzneimittel ab und führt dieses transdermal dem Körperteil 55 zu. Das Lösungsmittel verbleibt dabei im Speicherraum 25. Wenn der Arzneimittelvorrat mehr oder weniger vollständig aufgebraucht oder keine Arzneimittel-Zufuhr mehr erforderlich ist, kann die Einrichtung 1 vom Körperteil 55 entfernt werden. Des weiteren kann der Support 27 mitsamt den von ihm gehaltenen Teilen 29, 31, 33, 35, 37, 39, 41 vom Speicher 3 getrennt und für die nächste Benutzung mit einem anderen Speicher verbunden werden, dessen Speicherraum Arzneimittel enthält. Der vorher benutzte Speicher kann weggeworfen oder eventuell vollständig entleert, gereinigt, steril gemacht und für eine neue Benutzung gefüllt werden.

Für die Untersuchung der Abgabe eines Arzneimittels wurde eine ähnlich wie die Einrichtung 1 ausgebildete Versuchseinrichtung statt an einem Körperteil an einer Messvorrichtung befestigt. Mit dieser konnte das durch die Membran hindurchdiffundierte Arzneimittel in einer Wasser enthaltenden Zelle gesammelt und von Zeit zu Zeit die Menge des gesammelten Arzneimittels bestimmt werden. Die bei einem solchen Versuch durchgeführte Steuerung des Steuerelements und die daraus resultierende Abgaberate sind im Diagramm in der Fig. 7 veranschaulicht. Bei diesem Versuch enthielt der Speicherraum als Arzneimittel Salicylsäure, die vollständig in einem Öl gelöst war. Das Volumen der gespeicherten Lösung betrug dabei 2,5 cm³. Auf der zuunterst in der Fig. 7 ersichtlichen Abszisse ist die Zeit t in Minuten aufgetragen. Auf der Ordinate des sich inmittelbar über der Abszisse befindenden, unteren Teil-Diagramms ist der mit S bezeichnete Zustand des Steuerelements angegeben, wobei der Wert 0 dem Sperr-Zustand und der Wert 1 dem Freigabe-Zustand entspricht. Im oberen Teil-Diagramm ist auf der Ordinate die Abgaberate Q aufgetragen. Für die Ermittlung von Q wurde in Zeitabständen von 15 min die während den vergangenen 15 min aus dem Speicherraum gekommene und durch die Membran hindurchdiffundierte Menge Salicylsäure bestimmt.

Die im unteren Teil-Diagramm gezeichnete, rechteckige Steuerkurve 61 zeigt den für den Versuch gewählten, zeitlichen Verlauf des Zustands des Steuerelements 17. Dieses wurde beim Beginn der Messung im Zeitpunkt tₒ = 0 in den Freigabe-Zustand gebracht, während 30 min in diesem belassen, im Zeitpunkt t₁ in den Sperr-Zustand gebracht, während 150 min in diesem belassen, im Zeitpunkt t₂ wieder in den Freigabe-Zustand gebracht, während 120 min in diesem belassen und im Zeitpunkt t₃ wieder in den Sperr-Zustand gebracht. Die Messkurve 63 im oberen Teil-Diagramm zeigt den bei diesem Zustandsverlauf gemessenen, zeitlichen Verlauf der Abgaberate Q. Wie man sehen kann, nimmt die Abgabe im Verlauf der Freigabe-Zeitintervalle und auch während der Sperr-Zeitintervalle ab. Im oberen Teil-Diagramm ist noch gestrichelt eine Freigabe-Approximationsgerade 65 eingezeichnet, welche die Schwankungen der in den zwei aufeinander folgenden Freigabe-Zeitintervallen gemessenen Messwerte ausgleicht und das zwischen diesen Zeitintervallen liegende Sperr-Zeitintervall überbrückt. Die ebenfalls gestrichelt eingezeichnete Sperr-Approximationsgerade 67 gleicht analog die Schwankungen der in den zwei Sperr-Zeitintervallen gemessenen Messwerte aus und überbrückt das zwischen diesen Zeitintervallen liegende Freigabe-Zeitintervall. Ein Vergleich von zu gleichen Zeitpunkten gehörenden, durch die beiden Approcimationsgeraden 65 und 67 dargestellten Approximationswerten zeigt, dass die in der genannten Weise ausgeglichenen und angenäherten Abgaberaten im Sperr-Zustand ungefähr 15 bis 20% der sich im Freigabe-Zustand ergebenden Abgaberaten betragen. Es wurden auch Versuche durchgeführt, bei denen der Speicherraum noch ungelöste Salicylsäure enthielt und die Konzentration der gelösten Salicylsäure während der Abgabe konstant blieb. Bei diesen Versuchen betrugen die Abgaberaten im Sperr-Zustand ebenfalls ungefähr 15 bis 20% der sich im Freigabe-Zustand ergebenden Abgaberaten.

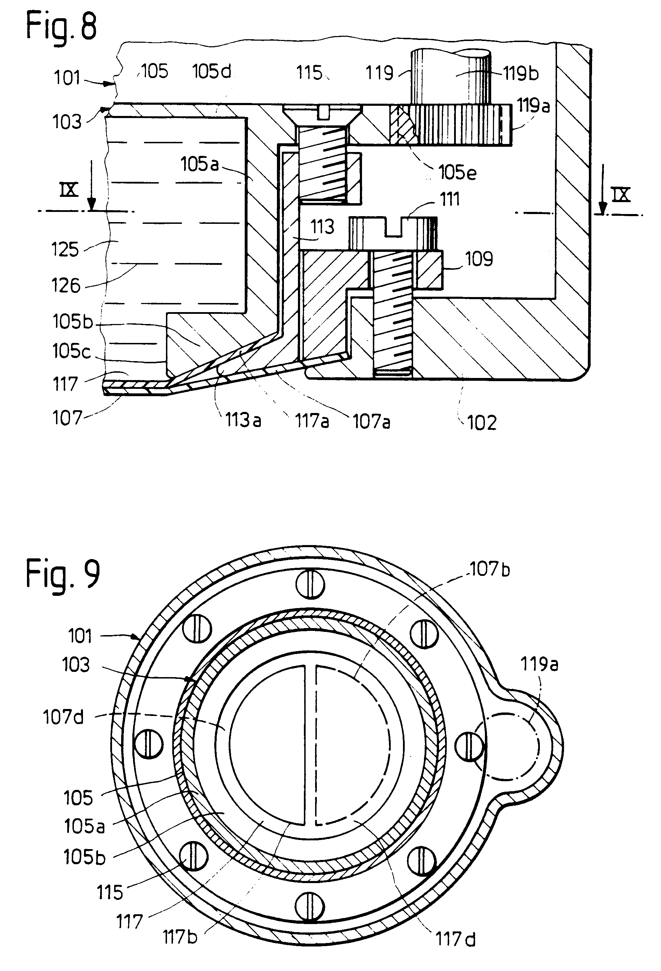

Die teilweise in der Fig. 8 sowie zudem in der Fig. 9 gezeichnete und als Ganzes mit 101 bezeichnete Einrichtung besitzt ein feststehendes, äusseres Gehäuse 102 und einen Speicher 103 mit einem Speichergehäuse 105, das im Gehäuse 102 um die gemeinsame Achse der beiden Gehäuse drehbar ist. Das Speichergehäuse 105 besitzt einen zylindeischen Mantel 105a, an dessen unteres Ende ein Wandabschnitt 105b mit einer konischen Aussenfläche anschliesst. Der Speicherbehälter 105 besitzt anstelle der ersten ebenen Wand 5c eine durch den inneren Rand des Wandabschnitts 105b begrenzte Öffnung 105c und ist also beim unteren Ende offen. Der Speicherbehälter 105 besitzt beim oberen Ende des Mantels 105a eine der zweiten Wand 5d des Speicherbehälters 5 entsprechende, radiale sowie ebene Wand 105d und zudem eine um seine Achse herum verlaufende Verzahnung 105e.

Eine Membran 107 liegt mit ihrem Randbereich 107a an einem konischen Flächenabschnitt des äusseren Gehäuses 102 an und ist mit einen äusseren Klemmring 109 sowie Schrauben 111 aufweisenden Befestigungsmitteln lösbar am äusseren Gehäuse 102 befestigt.

Ein innerer Klemmring 113 besitzt einen zwischen dem Mantel 105a des Speichergehäuses 105 sowie dem äusseren Klemmring 109 angeordneten Mantel und einen von diesem weg nach innen zwischen den Speichergehäuse-Wandabschnitt 105b und den Membran-Randabschnitt 107a hineinragenden Vorsprung 113a. Der innere Klemmring 113 ist mit Schrauben 115 am Speichergehäuse 105 gehalten und bildet zusammen mit diesen Befestigungsmittel zur lösbaren Befestigung eines Steuerelements 117 am Speichergehäuse 105. Das Steuerelement 117 ist durch eine flexible Folie gebildet und hat einen Randabschnitt 117a, der an der konischen Aussenfläche des Wandabschnitts 105b des Speichergehäuses 105 anliegt und durch den Vorsprung 113a des inneren Klemmrings 113 am Wandabschnitt 105b festgeklemmt wird.

Die Membran 107 und das Steuerelement 117 sind zum Beispiel aus Folien gebildet, die aus einem der in der Einleitung genannten, porösen Membranmaterialien bestehen. Die Membran 107 und die das Steuerelement 117 bildende Folie ist jedoch nur in einem ungefähr halbkreisförmigen Durchlassbereich 107b bzw. 117b für das Arzneimittel durchlässig. Die restlichen Bereiche der Membran 107 und des Steuerelements 117 oder mindestens der ebenen, kreiförmigen, sich in axialer Projektion innerhalb der Offnung 105c befindenden Abschnitte der Membran und des Steuerelements sind für das Arzneimittel undurchlässig und bilden einen Sperrbereich 107d bzw. 117d. Bei der Herstellung der Einrichtung 101 können die Poren der ursprünglich überall für das Arzneimittel durchlässigen Folien in den zu bildenden Sperrbereichen verschlossen und/oder abgedeckt werden. Dies kann zum Beispiel durch Aufbringen eines Verschlussmaterials - etwa durch Aufdampfen von Metall oder Aufsprühen eines Lacks - geschehen. Es besteht jedoch auch die Möglichkeit, dünne, porenfreie Folien als Verschlussmaterial auf die porösen Folien aufzukleben.

Die Aussenfläche des ebenen, kreisförmigen Abschnitts der Membran 107 bildet in der in der Fig. 8 gezeichneten Lage der Einrichtung 101 deren tiefstliegende Aussenfläche. Das Speichergehäuse 105 und das an ihm befestigte Steuerelement 117 werden durch den äusseren Klemmring 109, die Membran 107 und zusätzliche, nicht gezeichnete Lagermittel vorzugsweise derart radial sowie axial gelagert, dass das Steuerelement 117 bei allen möglichen Lagen der Einrichtung 101 und insbesondere des äussern Gehäuses 102 von dieser an der Membran 107 anliegt, aber leicht über diese gleiten kann. Ein drehbares Übertragungselement 119 besitzt ein mit der Verzahnung 105e des Speichergehäuses 105 in Eingriff stehendes Zahnrad 119a sowie eine fest mit diesem verbundene Welle 119b. Das Speichergehäuse 105 begrenzt zusammen mit dem Steuerelement 117 den Speicherraum 125, in dem das Speicher- und/oder Füllmaterial 126 gespeichert ist.

Bei der in den Figuren 8 sowie 9 gezeichneten Stellung des Speichergehäuses 105 und des an diesem befestigten Steuerelements 117 befindet sich das letztere in seinem Sperr-Zustand und bedeckt mit seinem Sperrbereich 117d den Durchlassbereich 107b der Membran 107, so dass fast kein Arzneimittel zum Durchlassbereich 107b gelangt. Wenn das Speichergehäuse 105 zusammen mit dem an ihm befestigten Steuerelement 117 um einen Zentriwinkel von 180° gedreht wird, gelangt das letztere in den Freigabe-Zustand, in welchem die beiden Durchlassbereiche 107b sowie 117b einander überdecken. Das im Speicher und/oder Füllmaterial 126 enthaltene, gelöste Arzneimittel kann dann durch die beiden Durchlassbereiche hindurchdiffundieren.

Die Welle 119a des Übertragungselements 119 ist radial sowie axial mit Lagermitteln eines nicht gezeichneten Supports gelagert, der lösbar mit dem äusseren Gehäuse 102 sowie dem Speichergehäuse 105 verbunden ist. Der nicht gezeichnete Support der Einrichtung 101 hält ähnlich wie der Support 27 der Einrichtung 1 einen elektrischen Motor, eine Elektronikvorrichtung und eine Batterie.

Es wurden auch Versuche mit einer weitgehend ähnlich wie die Einrichtung 101 ausgebildeten Versuchseinrichtung durchgeführt. Bei diesen Versuchen betrug die Abgaberate im Sperr-Zustand weniger als 10%, nämlich ungefähr oder höchstens 5% der sich im Freigabe-Zustand ergebenden Abgaberate. Bei der Einrichtung 101 ist also das Verhältnis zwischen den sich im Sperr-Zustand und im Freigabe-Zustand ergebenden Abgaberaten kleiner und damit günstiger als bei der Einrichtung 1. Dafür wird jedoch bei der Einrichtung 101 ein grösseres Drehmoment und mehr Energie zum Drehen des Steuerelements benötigt als bei der Einrichtung 1. Im übrigen kann die Einrichtung 101 in ähnlicher Weise benutzt werden wie die Einrichtung 1.

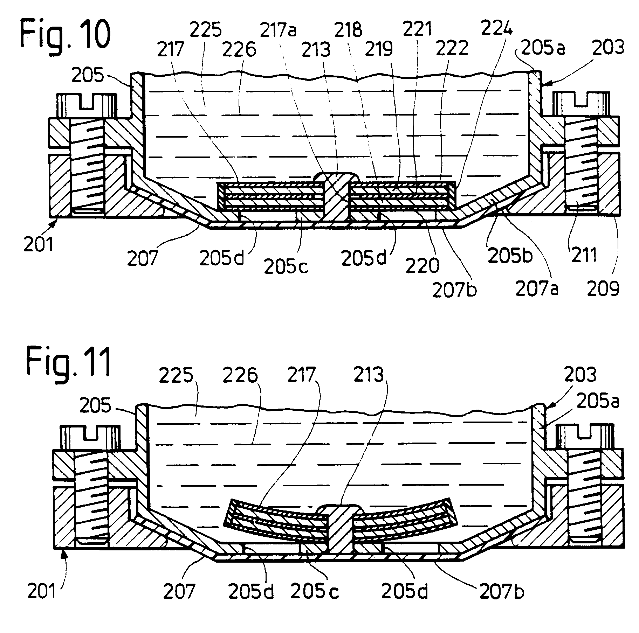

Die zum Teil in den Figuren 10 und 11 gezeichnete Einrichtung 201 besitzt einen Speicher 203 mit einem Speichergehäuse 205. Dieses hat einen Mantel 205a, einen Wandabschnitt 205b mit konischer Aussenfläche und eine mit diesem zusammenhängende, ebene Wand 205c. Diese besitzt mindestens ein durchgehendes Loch 205d und nämlich einige um ihr Zentrum herum verteilte Löcher 205d. Eine Membran 207 ist bei ihrem Randbereich 207a mit Hilfe von einem Klemmring 209 und von Schrauben 211 am Wandabschnitt 205b befestigt und liegt mit ihrem Durchlassbereich 207b an der ebenen Wand 205c an. Ein Steuerelement 217 mit der Form einer kreisförmigen Scheibe besitzt im Zentrum ein Loch 217a und ist mit einem dieses durchdringenden Befestigungselement 213 im Zentrum der ebenen Wand 205c befestigt, etwa angenietet.

Das Steuerelement 217 besteht aus einem mehrschichtigen Verbundkörper und besitzt mindestens ein scheibenförmiges piezoelektrisches Element und nämlich zwei solche Elemente 218, 219. Das Steuerelement 217 besitzt ferner drei schichtförmige Elektroden 220, 221, 222. Die unterste Elektrode 220 und die oberste Elektrode 222 sind elektrisch leitend miteinander und mit dem Massenanschluss einer nicht gezeichneten Elektronikvorrichtung verbunden. Die mittlere Elektrode 221 ist vom Befestigungselement 213 durch einen Ringspalt getrennt, beim Aussenrand durch eine Isolation 224 elektrisch gegen aussen isoliert und elektrisch leitend durch einen isolierten Leiter mit einem Steueranschluss der Elektronikvorrichtung verbunden. Der Speicherraum 225 enthält ein Speicher- und/oder Füllmaterial 226.

Wenn den Elektroden des Steuerelements 217 keine elektrische Spannung zugeführt wird, befindet sich das Steuerelement in seinem in der Fig. 10 gezeichneten Sperr-Zustand, in welchem es die Form einer ebenen Scheibe hat und die Löcher 205d abschliesst. Die Elektronikvorrichtung kann nun den Elektroden eine elektrische Gleichspannung zuführen, wobei zum Beispiel der mittleren Elektrode 221 eine gegenüber den beiden andern Elektroden 220, 222 positive Spannung zugeführt werden kann. Die beiden piezoelektrischen Elemente 218, 219 können derart angeordnet und polarisiert sein, dass die zugeführte elektrische Spannung eine radiale Dehnung des unteren Elements 218 und eine radiale Kontraktion des oberen Elements 219 bewirkt. Dadurch wird das Steuerelement 217 verformt, nämlich gemäss der Fig. 11 im Bereich der Löcher 205d von der Wand 205c weggebogen, so dass es in seinen Freigabe-Zustand gelangt.

Die Einrichtungen können noch auf andere Weisen geändert werden. Die Gehäuse 5, 102, 105, 205, und die Klemmringe 9, 109, 113, 209 können beispielsweise statt aus einem metallischen Material aus einem Kunststoff, wie zum Beispiel aus dem unter dem Handelsnamen DELRIN von der Firma Du Pont de Nemours erhältlichen Polymethylenoxid bestehen. Die Wanddicken der Gehäuse können dann nötigenfalls im Vergleich zu deren Durchmessern etwas grösser bemessen werden, als es in den verschiedenen Figuren gezeichnet ist. Wenn die Gehäuse und Klemmringe aus einem thermoplastischen Kunststoff bestehen, können die Ringe statt durch Schrauben durch eine Ultraschall-Schweissbindung miteinander verbunden werden. Die in axialer Richtung gemessene Dicke der Ringe kann dann im Vergleich zu deren Durchmesser eventuell kleiner bemessen werden als bei den Klemmringen 9, 109, 209. Zudem kann möglicherweise auch der Winkel, den der zwischen einem Gehäuse und einem Ring liegende Randbereich einer Membran mit deren ebenem Hauptabschnitt bildet, kleiner bemessen werden, ohne dass der Ring in axialer Richtung über die Membran vorsteht. Die Membranen können dann wie bei den anhand der verschiedenen Figuren beschriebenen Einrichtungen zwischen den Gehäusen und Klemmringen festgeklemmt werden. Eventuell können die Membranen jedoch ebenfalls mit den Gehäusen und Ringen verschweisst und/oder verklebt werden. Unter Umständen kann man die Membranen sogar ohne Ringe durch Schweissen und/oder Kleben an den Gehäusen befestigen. Dies kann ermöglichen, die Membranen ohne Abwinkeln oder Abbiegen ihrer Randbereiche zu befestigen, so dass die Membranen vollständig eben sein können.

Eventuell kann das Steuerelement 17 statt aus einem metallischen Material aus einem Kunststoff hergestellt werden. Ferner kann man die Sperrbereiche 17d des Steuerelements 17 statt durch federnde Zungen eventuell durch überall mit dem restlichen Steuerelement 17 zusammenhängende und mit diesen zusammen in einer Ebene liegende Abschnitte einer einstückigen Scheibe bilden.

Des weitern kann man die in sich geschlossene Zahnkränze bildenden Verzahnungen 17e, 105e eventuell durch Verzahnungen ersetzen, welche die Schwenkachse des betreffenden Steuerelements nur zum Teil umschliessen. Eventuell kann man die Verzahnung 17e des Steuerelements 17 sowie das Übertragungselement 19 sogar weglassen und das Steuerelement dafür fest mit einer zur Achse 15 koaxialen Welle verbinden. Diese kann dann durch eine im Zentrum der zweiten ebenen Wand 5d vorhandene, dichte Durchführung hindurch über eine oberhalb der Wand 5d angeordnete, entkuppelbare Kupplung mit einem vom Support 27 gehaltenen Getriebe verbunden sein. Der Support 27 kann statt durch Rastmittel durch Schrauben mit dem Speichergehäuse verbunden sein. Wenn die vorgängig erwähnte, das Übertragungselement 19 ersetzende Welle koaxial zum Mantel des Speichergehäuses angeordnet wird, besteht zudem die Möglichkeit, den Speichergehäuse-Mantel und den Support mit Gewinden zu versehen, die miteinander verschraubt werden können. Das ein Septum bildende Verschlusselement 27 kann ferner statt im Mantel in der zweiten ebenen Wand 5d des Speichergehäuses angeordnet werden. Des weiteren kann man das Steuerelement 107 mit zwei oder mehr um seine Achse herum verteilten Durchlassbereichen und der gleichen Anzahl Sperrbereiche versehen.

Die drehbaren Steuerelemente 17 der Einrichtungen 1 bzw. 101 können - wie es für das Steuerelement 17 anhand der Figur 7 erläutert wurde - intermittierend und schrittweise verschwenkt und dabei abwechselnd in den Freigabe-Zustand und in den Sperr-Zustand gebracht werden. Eventuell kann man jedoch auch vorsehen, ein drehbares Steuerelement während der ganzen Benutzungsdauer der Einrichtung gleichmässig zu drehen. Der zeitliche Verlauf der Arzneimittel-Abgabe kann dann bei einer zum Beispiel im allgemeinen gemäss der Einrichtung 1 ausgebildeten Einrichtung durch die Anzahlen, Anordnungen sowie Abmessungen der Durchlassbereiche 5i und Durchlasslöcher 17c und durch die Winkelgeschwindigkeit des Steuerelements 17 festgelegt werden. Mann kann dann zum Beispiel in der Wand 5c nur einen einzigen Durchlassbereich 5i und im drehbaren Steuerelement 17 nur ein einziges Durchgangsloch 17 oder mehrere Durchgangslöcher 17c vorsehen. Das bzw. jedes Durchgangsloch 17c kann sich dabei ungefähr über einen gleich grossen Zentriwinkel oder Bogen um die Achse 15 herum erstrecken wie der Durchlassbereich 5i. Stattdessen kann man jedoch auch vorsehen, dass sich das bzw. jedes Durchgangsloch 17 über einen wesentlich grösseren oder über einen wesentlich kleineren Zentriwinkel erstreckt als der Durchlassbereich 5i, so dass sich trotz der kontinuierlichen Drehung des Steuerelements mit konstanter Winkelgeschwindigkeit periodisch jeweils während einer gewissen Zeitdauer eine ungefähr konstante Arzneimittel-Abgaberate ergibt. Falls mehrere Durchlasslöcher 17c vorhanden sind, können diese gleiche Abmessungen besitzen und regelmässig verteilt sein. Man kann jedoch auch vorsehen, dass die Durchlasslöcher 17c sich über unterschiedliche Zentriwinkel erstrecken und/oder ungleichmässig um die Achse 15 herum verteilt sind. In einem solchen Fall wird dann bei einer mit einer konstanten Winkelgeschwindigkeit erfolgenden Drehung des Steuerelements um einen Winkel von 360° während zwei oder mehr unterschiedlich langen und/oder ungleichmässig verteilten Zeitintervallen Arzneimittel abgegeben. Statt eines einzigen Durchlassbereichs 5i und mehrer Durchlasslöcher 17c kann man selbstverständlich auch mehrere Durchlassbereiche 5i und nur ein einziges Durchlassloch 17c vorsehen. Des weiteren kann man sowohl mehrere Durchlassbereiche 5i als auch mehrere Durchlasslöcher 17c vorsehen, wobei die Anzahl der Durchlasslöcher 17c eventuell verschieden von der Anzahl der Durchlassbereiche 5i sein kann. Bei der Einrichtung 101 kann man in analoger Weise die Anzahlen, Abmessungen und Verteilungen der Durchlassbereiche 107b und 117b ändern.

Bei der Einrichtung 201 kann man die Wand 205c möglicherweise mit einer elastisch deformierbaren, alle Löcher 205d umschliessenden Dichtung versehen.

## Patentansprüche

1. Einrichtung zur Abgabe eines Arzneimittels, mit einem ein fliessfähiges Lösungsmittel und das mindestens zum Teil in diesem gelöste Arzneimittel enthaltenden Speicherraum (25, 125, 225), mit einer Membran (7, 107, 207), die mindestens einen für das Arzneimittel durchlässigen Durchlassbereich (7b, 107b, 207b) besitzt, und mit einem auf der dem Speicherraum (25, 125, 225) zugewandten Seite der Membran (7, 107, 207) angeordneten, verstellbaren und/oder verformbaren Steuerelement (17, 117, 217) zum Verändern des Zuganges des Arzneimittels vom Speicherraum (25, 125, 225) zum mindestens einen Durchlassbereich (7b, 107b, 207b) der Membran (7, 107, 207), gekennzeichnet durch
eine elektronische Schaltung (37) sowie durch diese elektrisch steuerbare Stellmittel zum Verstellen (19, 29, 31, 33, 35) bzw. Verformen (217) des Steuerelementes (17, 117, 217), um eine automatische Arzneimittelabgabe durch den mindestens einen Durchlassbereich (7b, 107b, 207b) der Membran (7, 107, 207) zu ermöglichen.

2. Einrichtung nach Anspruch 1, dadurch gekennzeichnet, dass das Steuerelement (17, 117) durch einen Schieber gebildet und parallel zu einem den bzw. jeden Durchlassbereich (7b, 107b) aufweisenden Abschnitt der Membran (7, 107) verstellbar ist.

3. Einrichtung nach Anspruch 1 oder 2, dadurch gekennzeichnet, dass das Steuerelement (7) um eine Achse (15) verschwenkbar ist, dass der Durchlassbereich (7b) der Membran (7) mit seiner dem Speicherraum (25) zugewandten Innenfläche (7d) an einer ebenen Wand (5c) anliegt, die mindestens ein durchgehendes Loch (5k) aufweist, und dass das Steuerelement (17) durch Verschwenken um die Achse (15) wahlweise in einen Sperr-Zustand und in einen Freigabe-Zustand gebracht werden kann, in dem es das bzw. jedes Loch (5k) der Wand (5c) abdeckt bzw. freigibt.

4. Einrichtung nach Anspruch 3, dadurch gekennzeichnet, dass die Wand (5c) mindestens zwei um die Achse (15) herum verteilte Durchlassbereiche (5i) mit je mindestens einem Loch (5k) der genannten Art aufweist, dass das Steuerelement (17) für jeden Durchlassbereich (5i) der Wand (5c) ein Durchlassloch (17c) und einen Sperrbereich (17d) aufweist und dass die Durchlasslöcher (17c) und Sperrbereiche (17d) des Steuerelements (17) derart ausgebildet sowie um die Achse (15) herum verteilt sind, dass jeder Sperrbereich (17d) des Steuerelements (17) in dessen Sperr-Zustand einen Durchlassbereich (5i) der Wand (5c) abdeckt und jedes Durchlassloch (17c) des Steuerelements (17) in dessen Freigabe-Zustand mindestens teilweise in Deckung mit einem Durchlassbereich (5i) der Wand (5c) steht.

5. Einrichtung nach Anspruch 4, dadurch gekennzeichnet, dass das Steuerelement (17) einen ebenen, jedes Durchlassloch (17c) begrenzenden Hauptabschnitt (17a) aufweist und dass der bzw. jeder Sperrbereich (17d) durch eine mit dem Hauptabschnitt (17a) zusammenhängende, federnde Zunge gebildet ist, die einen vom Hauptabschnitt (17a) weg zur Wand (5c) hin versetzten und federnd an dieser anliegenden Abschnitt hat.

6. Einrichtung nach Anspruch 2, dadurchgekennzeichnet, dass die Membran (107) aus einer Folie mit Poren besteht, die in dem bzw. jedem Durchlassbereich (107) der Membran (107) offen sind, dass die Membran (107b) mindestens einen für das Arzneimittel undurchlässigen Sperrbereich (107d) aufweist, in dem die Poren verschlossen sind, dass das Steuerelement (117) um eine Achse verschwenkbar ist und eine an der Membran (107) anliegende Folie mit Poren, mindestens einen für das Arzneimittel durchlässigen Durchlassbereich (117b) mit offenen Poren und mindestens einem fur das Arzneimittel undurchlässigen Sperrbereich (117d) mit verschlossenen Poren aufweist, dass das Steuerelement (117) durch Verschwenken um die genannte Achse wahlweise in einen Sperr-Zustand und in einen Freigabe-Zustand gebracht werden kann und dass der bzw. jeder Durchlassbereich (107b) der Membran (107) im Sperr-Zustand durch den bzw. einen Sperrbereich (117d) des Steuerelements (117) und im Freigabe-Zustand durch den bzw. einen Durchlassbereich (117b) des Steuerelements (117) bedeckt ist.

7. Einrichtung nach Anspruch 1, dadurch gekennzeichnet, dass die Stellmittel (19, 29, 31, 33, 35, 217) einen elektrischen Motor (31) umfassen.

8. Einrichtung nach Anspruch 1, dadurch gekennzeichnet, dass die Stellmittel ein verformbares, piezo-elektrisches Element (218, 219) umfassen, das mit dem Steuerelement (217) vereint ist.

9. Einrichtung nach Anspruch 1, dadurch gekennzeichnet, dass eine Batterie (41) zur Speisung der elektronischen Schaltung (37) und des Stellmittels (31, 217) vorgesehen ist und dass die elektronische Schaltung (37) programmiert ist, um die automatische Arzneimittelabgabe autonom ablaufen zu lassen.

10. Einrichtung nach Anspruch 9, dadurch gekennzeichnet, dass ein den Speicherraum (25, 125, 225) begrenzendes und das Steuerelement (17, 117, 217) haltendes Speichergehäuse (5, 105, 205) und ein lösbar mit dieser verbundener, die Elektronikvorrichtung (37) sowie die elektrisch mit dieser verbundene Batterie (41) haltender Support (27) vorhanden ist, der zusammen mit der Elektronikvorrichtung (37) und der Batterie (41) vom Speichergehäuse (5, 105, 205) wegnehmbar ist, wobei der Support (27) zum Beispiel auch noch einen zum Verstellen des Steuerelements (17, 117) dienenden Motor (31) hält, der ebenfalls zusammen mit dem Support (27) vom Speichergehäuse (5, 105) wegnehmbar ist.

11. Einrichtung nach Anspruch 1, dadurch gekennzeichnet, dass die elektronische Schaltung (37) derart ausgebildet ist, dass die automatische Arzneimittelabgabe in vorbestimmter Weise erfolgt.

## Claims

1. Device for delivering a medicament having a free-flowing solvent and a reservoir space (25, 125, 225) containing medicament at least in part dissolved in said solvent, which has a membrane (7, 107, 207) with at least one medicament permeable area (7b, 107b, 207b) and provided with an adjustable and/or deformable control element (17, 117, 217) disposed on the side of the membrane (7, 107, 207) facing the reservoir space (25, 125, 225) to change the entry of the medicament from the reservoir space (25, 125, 225) to at least one permeable area (7b, 107b, 207b) of the membrane (7, 107, 207), characterized by an electronic circuit (37) as well by this electrically controllable adjustment means (19, 29, 31, 33, 35) for respectively adjusting or deforming (217) the control element (17, 117, 217), so as to allow for an automatic medicament delivery through said at least one permeable area (7b, 107b, 207b) of said membrane (7, 107, 207).

2. Device according to claim 1, characterized in that the control element (17, 117) is formed by a sliding member and is adjustable parallel to a section of the membrane (7, 107) having one or each permeable area (7b, 107b).

3. Device according to claim 1 or 2, characterized in that the control element (7) can be tilted about an axis (15), that the permeable area (7b) of the membrane (7) abuts with its inner surface (7d) facing the reservoir area (25) against a planar wall (5c), which has at least one through hole (5k) and that by tilting about the axis (15) the control element (17) can be brought either into a blocked state or into a release state in which it covers or releases the or each hole (5k) of the wall (5c).

4. Device according to claim 3, characterized in that the wall (5c) has at least two permeable areas (5i) distributed about the axis (15) each with at least one hole (5k) of the cited type, that the control element (17) has a permeable hole (17c) and a blocking area (17d) for each permeable area (5i) of the wall (5c), and that the permeable holes (17c) and the blocking areas (17d) of the control element (17) are so devised and distributed about the axis (15) that each blocking area (17d) of the control element (17) in the blocking state of the latter covers one permeable area (5i) of the wall (5c) and each permeable hole (17c) of the control element (17) in its release state at least partially overlaps the wall (5c) with one permeable area (5i).

5. Device according to claim 4, characterized in that the control element (17) has a planar main section (17a) limiting each permeable hole (17c) and that the or each blocking area (17d) is formed by one elastic tongue associated with the main section (17a) which has a section disposed away from the wall (5c) and lying in resilient manner thereagainst.

6. Device according to claim 2, characterized in that the membrane (107) consists of a foil with pores, which are open in the or each permeable area (107b) of the membrane (107), that the membrane (107) has at least one blocking area (107d) impermeable to the medicament in which the pores are closed, that the control element (117) can be tilted about an axis and has at least one permeable area (117b) with open pores permeable to the medicament and at least one impermeable blocking area (117d) with closed pores impermeable to the medicament, that the control element (117) can be brought selectively either closed state by tilting about the said axis and brought into a release state and that the or each permeable area (107b) of the membrane (107) in the blocked state is covered by the or one blocking area (117d) of the control element (117) and is covered in the release state by the or one permeable area (117b) of the control element (117).

7. Device according to claim 1, characterized in that the adjusment means (19, 29, 31, 33, 35, 217) comprise an electric motor (31).

8. Device according to claim 1, characterized in that the adjustment means comprise a deformable piezo-electric element (218, 219) which is united with the control element (217).

9. Device according to claim 1, characterized in that a battery (41) is provided for supplying the electronic circuit (37) and the adjustment means (31, 217) and in that the electronic circuit (37) is programmed to let the automatic medicament delivery operate in an autonomous manner.

10. Device according to claim 9, characterized in that a reservoir housing (5, 105, 205) is provided limiting the reservoir space (25, 125, 225) and retaining the control element (17, 117, 217) and a support (27) detachably associated therewith which holds the electronic device (37) as well as the battery (41) electrically associated therewith, which is removable from the reservoir housing (5, 105, 205) together with the electronic device (37) and the battery (41), whereby the support (27) for example also holds a motor (31) serving to adjust the control element (17, 117) which is also removable from the reservoir housing (5, 105) together with the support (27).

11. Device according to claim 1, characterized in that the electronic circuit (37) is realized in such a manner that the automatic medicament delivery operates in a predetermined manner.

## Revendications

1. Dispositif de délivrance d'un médicament ayant un réservoir (25, 125, 225) contenant un solvant coulant et le médicament au moins partiellement dissous dans celui-ci, une membrane (7, 107, 207) comprenant au moins une région perméable (7b, 107b, 207b) oui est perméable au médicament et un élément de commande (17, 117, 217) ajustable et/ou déformable, agencé sur la face de la membrane (7, 107, 207) en regard du réservoir (25, 125, 225), pour varier l'accès du médicament du réservoir (25, 125, 225) à au moins une région perméable (7b, 107b, 207b) de la membrane (7, 107, 207), caractérisé par un circuit électronique (37) et par des moyens de positionnement commandés électriquement par ce dernier pour ajuster (19, 29, 31, 33, 35), respectivement déformer (217) l'élément de commande (17, 117, 217) afin de permettre une délivrance automatique de médicament à travers ladite au moins une région perméable (7b, 107b, 207b) de la membrane (7, 107, 207).

2. Dispositif selon la revendication 1, caractérisé en ce quel'élément de commande (17, 117) est formé par une vanne et est ajustable parallèlement à une section de la membrane (7, 107) présentant la, respectivement chaque région perméable (7b, 107b).

3. Dispositif selon la revendication 1 ou 2, caractérisé en ce que l'élément de commande (7) est pivotable autour d'un axe (15), en ce que la région perméable (7b) de la membrane (7) avec sa surface intérieure (7d) en regard du réservoir (25) est adjacente à une paroi plane (5c) qui présente au moins un trou traversant (5k), et en ce que l'élément de commande peut être amené par pivotement autour de l'axe (15) sélectivement dans un état d'obturation et dans un état de libération par le fait qu'il recouvre, respectivement libère le, respectivement chaque trou (5k) de la paroi (5c).

4. Dispositif selon la revendication 3, caractérisé en ce que la paroi (5c) présente au moins deux régions de passage (5i) distribuées autour de l'axe (15) et ayant chacune au moins un trou (5k) de la sorte mentionnée, en ce que l'élément de commande (17) présente pour chaque région de passage (5i) de la paroi (5c) un trou de passage (17c) et une région d'obturation (17d), et en ce que les trous de passage (17c) et les régions d'obturation (17d) de l'élément de commande (17) sont formés et distribués autour de l'axe (15) de manière que chaque région d'obturation (17d) de l'élément de commande (17) recouvre une région de passage (5i) de la paroi (5c) dans son état d'obturation et que chaque trou de passage (17c) de l'élément de commande (17) est situé au moins partiellement en regard d'une région de passage (5i) de la paroi (5c).

5. Dispositif selon la revendication 4, caractérisé en ce que l'élément de commande (17) présente une section principale plane (17a) délimitant chaque trou de passage (17c), et en ce que la, respectivement chaque région d'obturation (17d) est formée par une langue élastique reliée continûment avec la section principale (17a), cette langue ayant une section décalée relativement à la section principale (17a) en direction de la paroi (5c) et disposée élastiquement contre celle-ci.

6. Dispositif selon la revendication 2, caractérisé en ce que la membrane (107) consiste en une feuille avec des pores qui sont ouverts dans la, respectivement chaque région perméable (107) de la membrane (107), en ce que la membrane (107b) présente une région d'obturation (107d) imperméable pour le médicament dans laquelle les pores sont fermés, en ce que l'élément de commande (117) est pivotable autour d'un axe et présente une feuille avec des pores adjacente à la membrane (107), au moins une région perméable (117b) avec des pores ouverts qui est perméable au médicament et au moins une région d'obturation (117d) avec des pores fermés qui est imperméable pour le médicament, en ce que l'élément de commande (117) peut être amené par pivotement autour dudit axe sélectivement dans un état d'obturation et dans un état de libération, et en ce que la, respectivement chaque région perméable (107b) de la membrane (107) est recouvert dans l'état d'obturation par la, respectivement une région d'obturation (117d) de l'élément de commande (117) et dans l'état de libération par la, respectivement une région perméable (117b) de l'élément de commande (117).

7. Dispositif selon la revendication 1, caractérisé en ce que les moyens de positionnement (19, 29, 31, 33, 35, 217) comprennent un moteur électrique (31).

8. Dispositif selon la revendication 1, caractérisé en ce que les moyens de positionnement comprennent un élément piézo-électrique déformable (218, 219) qui est uni avec l'élément de commande (217).

9. Dispositif selon la revendication 1, caractérisé en qu'il est prévu une batterie (41) pour alimenter le circuit électronique (37) et le moyen de positionnement (31, 217), et en ce que le circuit électronique (37) est programmé pour laisser fonctionner de manière autonome la délivrance automatique de médicament.

10. Dispositif selon la revendication 9, caractérisé en ce qu'il est prévu un boîtier de réservoir (5, 105, 205) délimitant le réservoir (25, 125, 225) et maintenant l'élément de commande (17, 117, 217) et un support (27) lié de manière amovible au boîtier de réservoir et maintenant l'installation électronique (37) ainsi que la batterie (41) reliée électriquement à celle-ci, ce support ensemble avec l'installation électronique (37) et la batterie (41) étant détachable du boîtier de réservoir (5, 105, 205), ce support (27) maintenant aussi par exemple encore un moteur (31) servant à ajuster l'élément de commande (17, 117) qui est également avec le support (27) détachable du boîtier de réservoir (5, 105).

11. Dispositif selon la revendication 1, caractérisé en ce que le circuit électronique (37) est agencé de manière que la délivrance automatique de médicament se produit d'une façon prédéterminée.
